# EUROPEAN PATENT APPLICATION

(11) **EP 4 206 332 A1**
(43) Date of publication of application: **05.07.2023**
(21) Application number: 21861130.9
(22) Date of filing: 30.07.2021
(51) Int. Cl.: C12Q 1/6837, C12M 1/00, C12M 1/34, C12N 15/09, G01N 21/64, G01N 33/53, G01N 33/543

(54) **TARGET MEASUREMENT METHOD, DEVICE FOR TARGET MEASUREMENT, TARGET MEASUREMENT APPARATUS, AND KIT FOR TARGET MEASUREMENT**

(30) Priority: 31.08.2020 JP 2020145840
(71) Applicant: Yokogawa Electric Corporation, Musashino-shi Tokyo 180-8750 (JP)
(72) Inventor: MIYAUCHI, Yuki, Musashino-shi, Tokyo 180-8750 (JP); TADENUMA, Takashi, Musashino-shi, Tokyo 180-8750 (JP); TAGUCHI, Tomoyuki, Musashino-shi, Tokyo 180-8750 (JP)
(74) Representative: Winter, Brandl - Partnerschaft mbB
(86) International application number: PCT/JP2021/028455
(87) International publication number: WO 2022/044702

(57) **Abstract**

A target measurement method for measuring a target contained in a sample, includes, when a solution containing a light-absorbing substance that absorbs excitation light that excites a fluorescent molecule or fluorescence emitted from the fluorescent molecule is in contact with a solid-phase surface of a substrate that is provided with a bound product of the target and a capture molecule specifically binding to the target which is modified with a fluorescent molecule, measuring a fluorescence obtained by irradiation of the excitation light from an opposite side to the solid-phase surface of the substrate from the opposite side to the solid-phase surface of the substrate.

## Description

### [Technical Field]

The present invention relates to a target measurement method, a target measurement device, a target measurement apparatus, and a target measurement kit.

### [Background Art]

A method using a DNA microarray (a detection probe having a sequence complementary to a specific nucleic acid sequence provided on a solid-phase surface of a substrate or the like) is widely known as a method for measuring a target that has a specific nucleic acid sequence contained in a sample. This method is a method of measuring a target by utilizing a property that the target contained in a sample added to the DNA microarray is collected by the detection probe of the DNA microarray according to a hybridization reaction. In this method, in addition to whether the target is included in the sample, it is possible to measure the amount of the target included in the sample. The following Non-Patent Documents 1 and 2 and Patent Document 1 disclose conventional measurement methods for measuring a target using a DNA microarray.

### [Citation List]

### [Non-Patent Document]

[Non-Patent Document 1]
   Koichi HITRAYAMA et al., Development of DNA Chip for detection of UGT1A1 Polymorphisms, Toyo Kohan Vol. 38, 51-56
[Non-Patent Document 2] Vivian G. C., et al. Making and reading microarrays, Nature genetics supplement 21, 15-19 (1999)

### [Patent Document]

[Patent Document 1]
   Japanese Unexamined Patent Application Publication No. 2015-43702

### [Summary of Invention]

### [Technical Problem]

Incidentally, the nucleic acid sequence measurement methods of Non-Patent Documents 1 and 2 above requires a washing operation to remove uncollected targets, and there is a problem that the measurement accuracy may deteriorate with this washing operation. In addition, although the nucleic acid sequence measurement method of Patent Document 1 does not require the washing operation as in Non-Patent Documents 1 and 2, offset light emitted from a sample solution supplied to the DNA microarray becomes noise, and thus there is a problem that the measurement accuracy may deteriorate.

The present invention has been made in view of the circumstances described above, and an object thereof is to provide a target measurement method, a target measurement device, a target measurement apparatus, and a target measurement kit that can improve the measurement accuracy of a target included in a sample as compared with the conventional art.

### [Solution to Problem]

The present invention has adopted the following configuration to achieve the object described above.
[1] A target measurement method for measuring a target contained in a sample, may include: when a solution containing a light-absorbing substance that absorbs excitation light that excites a fluorescent molecule or fluorescence emitted from the fluorescent molecule is in contact with a solid-phase surface of a substrate that is provided with a bound product of the target and a capture molecule specifically binding to the target which is modified with a fluorescent molecule, measuring a fluorescence obtained by irradiation of the excitation light from an opposite side to the solid-phase surface of the substrate from the opposite side to the solid-phase surface of the substrate.
[2] The target measurement method according to [1], may further include: modifying the target with the fluorescent molecule; and obtaining the bound product by binding the target to the capture molecule immobilized on the solid-phase surface.
[3] The target measurement method according to [1], may further include: modifying the capture molecule with the fluorescent molecule; and obtaining the bound product by binding the capture molecule to the target immobilized on the solid-phase surface.
[4] The target measurement method according to [1], may further include: modifying the capture molecule with the fluorescent molecule; and obtaining the bound product by binding the target to the capture molecule immobilized on the solid-phase surface.
[5] The target measurement method according to [1], may further include: obtaining the bound product by supplying either one of the target and the capture molecule immobilized on the solid-phase surface and the fluorescent molecule to the other of the target and the capture molecule.
[6] The target measurement method according to any one of [1] to [5], in which the target is a nucleic acid with a specific nucleic acid sequence, the capture molecule is a detection probe having a sequence complementary to the specific nucleic acid sequence, and the target is subjected to a hybridization reaction with the detection probe to obtain the bound product.
[7] A target measurement device used when a target contained in a sample is measured, may include: a substrate on which either one of the target and a capture molecule specifically binding to the target is immobilized on a solid-phase surface; and a container to which a light-absorbing substance that absorbs excitation light that excites a fluorescent molecule that modify a bound product of the target and the capture molecule or fluorescence emitted from the fluorescent molecule is added, and which is capable of holding a solution containing the other of the target and the capture molecule and the light-absorbing substance while in contact with the solid-phase surface of the substrate.
[8] The target measurement device according to [7], in which the capture molecule is immobilized on the solid-phase surface, and a solution containing the target modified with the fluorescent molecule is supplied to the container.
[9] The target measurement device according to [7], in which the target is immobilized on the solid-phase surface, and a solution containing the target modified with the fluorescent molecule is supplied to the container.
[10] The target measurement device according to [7], in which the capture molecule, which is modified with the fluorescent molecule, is immobilized on the solid-phase surface, and a solution containing the target is supplied to the container.
[11] The target measurement device according to [7], in which either one of the target and the capture molecule is immobilized on the solid-phase surface, and a solution containing the other of the target and the capture molecule and the fluorescent molecule binding to a bound body of the target and the capture molecule is supplied to the container.
[12] The target measurement device according to any one of [7] to [11], in which the target is a target having a specific nucleic acid sequence, and the capture molecule is a detection probe having a sequence complementary to the specific nucleic acid sequence.
[13] A target measurement apparatus may include: the target measurement device according to any one of [7] to [12]; and a fluorescence reading device configured to measure an amount of fluorescence from the target measurement device.
[14] A target measurement kit used when a target contained in the sample is measured, may include: a substrate on which either one of the target and a capture molecule specifically binding to the target is immobilized on a solid-phase surface, a container capable of holding a solution containing the other of the target and the capture molecule while in contact with the solid-phase surface of the substrate, and a light-absorbing substance configured to absorb excitation light that excites a fluorescent molecule modifying a bound product of the target and the capture molecule or fluorescence emitted from the fluorescent molecule.
[15] The target measurement kit according to [14], in which the light-absorbing substance is added to the container in advance.
[16] The target measurement kit according to [14] or [15], in which the capture molecule is immobilized on the solid-phase surface, and a solution containing the target modified with the fluorescent molecule and the light-absorbing substance is held in the container.
[17] The target measurement kit according to [14] or [15], in which the target is immobilized on the solid-phase surface, and a solution containing the capture molecule modified with the fluorescent molecule and the light-absorbing substance is held in the container.
[18] The target measurement kit according to [14] or [15], in which the capture molecule modified with the fluorescent molecule is immobilized on the solid-phase surface, and a solution containing the target and the light-absorbing substance is held in the container.
[19] The target measurement kit according to [14] or [15], in which either one of the target and the capture molecule is immobilized on the solid-phase surface, and a solution containing the other of the target and the capture molecule, the fluorescent molecule binding to a bound body of the target and the capture molecule, and the light-absorbing substance is held in the container.
[20] The target measurement kit according to any one of [14] to [19], in which the target is a target having a specific nucleic acid sequence, and the capture molecule is a detection probe having a sequence complementary to the specific nucleic acid sequence.

### [Advantageous Effects of Invention]

According to the target measurement method, the target measurement device, the target measurement apparatus, and the target measurement kit of the present invention, there is an effect that the measurement accuracy of a target included in a sample can be improved as compared with the conventional art.

### [Brief Description of Drawings]

FIG. 1 is a diagram which shows a method of modifying a target using a fluorescent molecule, immobilizing a DNA probe on a solid-phase surface of a substrate, and binding the target modified using the fluorescent molecule to the DNA probe immobilized on the solid-phase surface.
FIG. 2 is a diagram which shows a method of modifying a DNA probe with a fluorescent molecule, immobilizing a target on a solid-phase surface of a substrate, and binding the DNA probe modified with the fluorescent molecule to the target immobilized on the solid-phase surface.
FIG. 3 is a diagram which shows a method of immobilizing a DNA probe (a donor fluorescent probe) modified with a fluorescent molecule and a quenching molecule (a quenching probe) that specifically binds to the DNA probe on a solid-phase surface of a substrate, and binding a target to the donor fluorescent probe.
FIG. 4 is a diagram which shows an example of a configuration of a target measurement device of the present invention.
FIG. 5 is a flowchart which shows an example of an operation procedure for detecting a target using the target measurement device of the present invention.
FIG. 6 is a diagram which shows an example of a configuration of a target measurement apparatus of the present invention.
FIG. 7 is a graph which shows a relationship between concentration of a fluorescent molecule in a solution and a light intensity of background light when a light-absorbing substance of Example 1 is added and when it is not added.
FIG. 8 is a diagram which shows a spot image of a spot where synthetic DNA modified using fluorescent molecule is immobilized on a substrate, obtained by a fluorescence reading device when the light-absorbing substance of Example 1 is added and when it is not added.
FIG. 9 is a diagram which shows a relationship between spot light intensity and background light when a Cy3 (registered trademark) molecule concentration of Example 1 is 30 nM.
FIG. 10 is a diagram which shows the relationship between spot light intensity and background light when a light-absorbing substance of Example 2 is added and when it is not added.

### [Description of Embodiments]

Hereinafter, a target measurement method, a target measurement device, a target measurement apparatus, and a target measurement kit according to embodiments of the present invention will be described in detail with reference to the drawings. In the following description, first, an outline of the embodiments of the present invention will be described, and then details of the embodiments of the present invention will be described.

### [Outline]

Embodiments of the present invention make it possible to improve measurement accuracy of a target contained in a sample as compared to the prior art. In a method disclosed in Non-Patent Document 1 described above, a fluorescence-modified PCR product obtained by performing PCR on a DNA sample using fluorescence-modified primers is added to a DNA microarray, and a hybridization reaction is performed thereon to detect the target in the sample. A method disclosed in Non-Patent Document 2 described above involves detecting the target in the sample by causing a hybridization reaction between a target immobilized on the microarray and a fluorescence-modified fluorescent probe.

The method disclosed in Patent Document 1 described above involves measuring a target using a nucleic acid sequence measurement device (DNA microarray) provided with, as detection probes, a fluorescent probe to which a fluorescent molecule is added, and a quenching probe to which a quenching molecule for quenching fluorescence of the fluorescent molecule is added. In this method, it is possible to measure a target without addition of a fluorescent molecule to the target or washing of a DNA microarray (washing for removing uncollected targets, and the like). In this method, in a nucleic acid sequence measurement device that measures the presence or absence and the amount of a specific nucleic acid using the DNA microarray, when a target is not present, a donor fluorescent probe and a quenching probe, which are independent from each other, maintain their binding via a binding unit, and the fluorescence of the fluorescent molecule is quenched by the quenching molecule. When a target is supplied, the target binds to a detection unit, the binding between the donor fluorescent probe and the quenching probe via the binding unit is eliminated, the quenching molecule is separated from the donor fluorescent molecule, and thereby the donor fluorescent molecule emits fluorescence. By using this nucleic acid sequence measurement device, the target contained in the sample can be measured.

However, the nucleic acid sequence measurement method of Non-Patent Document 1 described above requires a washing operation to remove uncollected targets, and a reacted target is peeled off by the washing operation, which may result in a decrease in the quantification of the target and deterioration in the lower limit of detection. Similarly, the nucleic acid sequence measurement method of Non-Patent Document 2 also requires a washing operation to remove the uncollected probe, and a reacted probe is peeled off by the washing operation, which may result in a decrease in the quantification of the target and a deterioration in lower limit of detection.

In addition, in the nucleic acid sequence measurement methods of Non-Patent Document 1 and Non-Patent Document 2, a sample may be mixed between adjacent wells on the microarray due to the washing operation required in the method, and the target may not be detected accurately.

In Patent Document 1 described above, it is possible to measure a target without washing the DNA microarray (washing to remove uncollected targets, and the like). However, nucleic acid samples extracted from a specimen including organisms and microorganisms as a target preparation often contain residual molecules such as proteins and sugars derived from the specimen. For this reason, the nucleic acid sample solution emits fluorescence when a fluorescence image is acquired, which is a factor that increases the light intensity of background light, and offset light is emitted from the sample solution supplied to the nucleic acid sequence measurement device. Such offset light becomes noise and causes the measurement accuracy to deteriorate. For example, if a target is very small, the fluorescence emitted from the sample solution supplied to the nucleic acid sequence measurement device will also be weak, and target measurement cannot be performed if this weak fluorescence is buried in the offset light.

### [Embodiment]

A target measurement method of the present embodiment involves measuring fluorescence obtained by irradiation of excitation light that excites the fluorescent molecule from an opposite side to the solid-phase surface of the substrate from the opposite side to the solid-phase surface of the substrate while a solution containing the excitation light that excites the fluorescent molecule or a light-absorbing substance (hereinafter, simply referred to as a "light-absorbing substance") that absorbs the fluorescence emitted from the fluorescent molecule is in contact with the solid-phase surface of the substrate provided with a bound product of a target and a capture molecule specifically binding to the target (hereinafter, simply referred to as a "capture molecule"), which are modified with fluorescent molecules. This makes it possible to improve the measurement accuracy of a target contained in a sample more than in the prior art. In addition, the washing operation of a substrate such as the DNA microarray is not necessary, and the effects of decrease in quantification and deterioration in the lower limit of detection based on the washing operation can be eliminated. In addition, the fluorescence of the sample solution derived from the specimen can be reduced, and weak light can be measured.

The target measurement method of the present embodiment is a target measurement method for measuring a target contained in a sample, and examples of a method of obtaining a bound product of the target and a capture molecule specifically binding to the target, which are modified with fluorescent molecules, include, for example, a method of obtaining the bound product by modifying the target with the fluorescent molecule and binding the target to the capture molecule immobilized on the solid-phase surface, a method of obtaining the bound product by modifying the capture molecule with the fluorescent molecule and binding the capture molecule to the target immobilized on the solid-phase surface, a method of obtaining the bound product by modifying the capture molecule with the fluorescent molecule and binding the target to the capture molecule immobilized on the solid-phase surface, a method of obtaining the bound product by supplying either one of the target and the capture molecule, which are immobilized on the solid-phase surface, and the fluorescent molecule to the other of the target and the capture molecule, and the like.

Examples of the method of obtaining the bound product by binding the target to the capture molecule modified with the fluorescent molecule immobilized on the solid-phase surface include, for example, a method of immobilizing a capture molecule modified with a fluorescent molecule and a quenching molecule specifically binding to the capture molecule modified with a quenching substance on a solid-phase surface such that the fluorescent molecule modifying the capture molecule is quenched by a quenching substance modifying the quenching molecule, preventing, when a target is not present, fluorescence from being generated even if a fluorescent molecule is excited by excitation light, and obtaining, when a target is present, a bound product by binding the target to the capture molecule. When the target binds to the capture molecule, the quenching substance that modifies the quenching molecule moves away from the fluorescent molecule that modifies the capture molecule, and thereby fluorescence is generated from the capture molecule immobilized on the solid-phase surface.

Examples of a method of obtaining the bound product by supplying either one of the target and the capture molecule immobilized on the solid-phase surface and the fluorescent molecule to the other of the target and the capture molecule include, for example, a method of obtaining a bound product by immobilizing either one of a nucleic acid having a specific nucleic acid sequence and a nucleic acid probe having a nucleic acid sequence complementary to the specific nucleic acid sequence on the solid-phase surface, and supplying the other of the nucleic acid and the nucleic acid probe and an intercalator bound to a bound body of the nucleic acid and the nucleic acid probe, which is modified with a fluorescent molecule, to the solid-phase surface.

A target is not particularly limited as long as it is an object to be detected in a sample, but examples thereof include nucleic acids such as DNA and RNA, peptides, proteins, and the like. Examples of a capture molecule that specifically binds to the target include a detection probe that hybridizes with a nucleic acid, an antibody or an antibody fragment that specifically binds to an antigen such as a peptide and a protein, an aptamer that specifically binds to a nucleic acid, and the like. Either a polyclonal antibody or a monoclonal antibody can be used as the antibody, and a monoclonal antibody is preferred. Examples of the antibody fragment include, for example, F(ab')₂, F(ab)₂, Fab', Fab, Fv, scFv, mutants of these, fusion proteins or fusion peptides containing an antibody portion, and the like. In addition, the target may be an antibody or antibody fragment, and the capture molecule may be an antigen such as a peptide or protein that specifically binds to the antibody or antibody fragment.

Examples of a combination of a target and a capture molecule that specifically binds to the target include, for example, a combination of a nucleic acid having a specific nucleic acid sequence as a target and a detection probe having a sequence complementary to the specific nucleic acid sequence as a capture molecule, a combination of an antigen as a target and an antibody or antibody fragment that specifically binds to the antigen as a capture molecule, and the like. When a target is a nucleic acid having a specific nucleic acid sequence and a capture molecule is a detection probe having a sequence complementary to the specific nucleic acid sequence, the nucleic acid that is the target binds to the detection probe that is the capture molecule by a hybridization reaction.

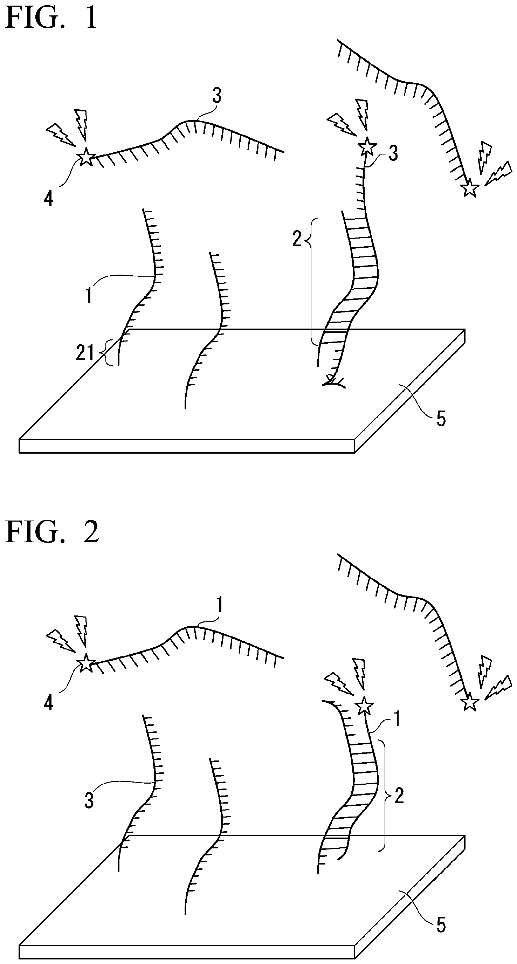

FIG. 1 shows a specific example of the method of modifying the target with the fluorescent molecule and obtaining the bound product by binding the target to the capture molecule immobilized on the solid-phase surface. In FIG. 1, the target 3 is DNA having a specific nucleic acid sequence, the DNA is modified with a fluorescent molecule 4, and the capture molecule is a DNA probe 1 having a detection sequence 2, which is a nucleic acid sequence complementary to the specific nucleic acid sequence of the target 3. The DNA probe 1 is immobilized on a DNA microarray 5 which is a substrate via a linker 21. The target 3 modified with the fluorescent molecule 4 binds to the DNA probe 1 immobilized on the DNA microarray 5 by a hybridization reaction, and fluorescence is emitted by exciting the fluorescent molecule 4 modifying the target 3 with excitation light.

FIG. 2 shows a specific example of the method of modifying the capture molecule with the fluorescent molecule and binding the capture molecule to the target immobilized on the solid-phase surface to obtain the bound product. In FIG. 2, the target 3 is DNA having a specific nucleic acid sequence, and the capture molecule is a DNA probe 1 having a detection sequence 2 that is a nucleic acid sequence complementary to the specific nucleic acid sequence of the target 3. The DNA probe 1 is modified with the fluorescent molecule 4, and the target 3 is immobilized on the DNA microarray 5 which is a substrate. The DNA probe 1 modified with the fluorescent molecule 4 binds to the target 3 immobilized on the DNA microarray 5 according to the hybridization reaction, and fluorescence is emitted by exciting the fluorescent molecule 4 modifying the DNA probe 1 with excitation light.

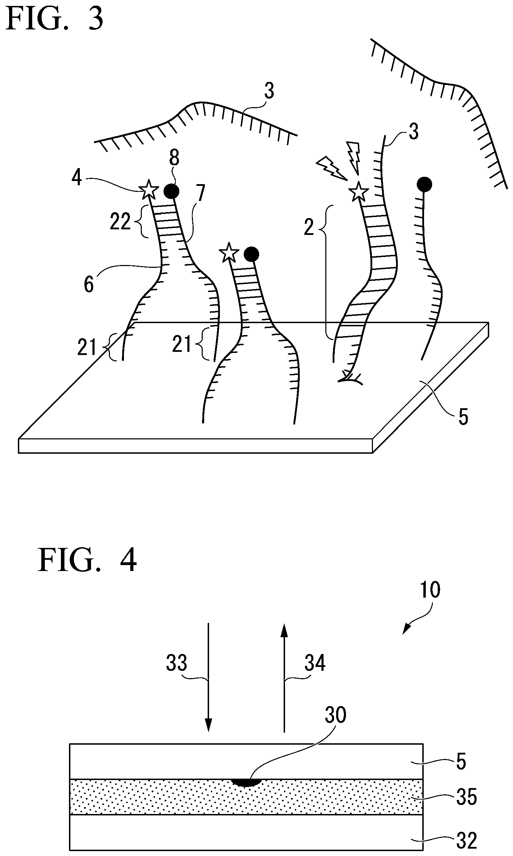

FIG. 3 shows a specific example of the method of modifying the capture molecule with the fluorescent molecule and binding the target to the capture molecule immobilized on the solid-phase surface to obtain the bound product. In FIG. 3, the target 3 is DNA having a specific nucleic acid sequence, and the capture molecule is a donor fluorescent probe 6 having a detection sequence 2 that is a nucleic acid sequence complementary to the specific nucleic acid sequence of the target 3, modified with the fluorescent molecule 4. The donor fluorescent probe 6 is immobilized on the DNA microarray 5 that is a substrate via the linker 21, and the quenching probe 7 having a sequence complementary to the nucleic acid sequence of the donor fluorescent probe 6 modified with the quenching substance 8 is immobilized on the DNA microarray 5 which is a substrate to hybridize with the donor fluorescent probe 6 using the binding unit 22. When the target 3 is not present, the fluorescent molecule 4 is quenched by the quenching substance 8, and fluorescence is not generated even if the fluorescent molecule 4 is excited with excitation light. When the target 3 is present, the target 3 binds to the detection sequence 2 of the donor fluorescent probe 6 modified with the fluorescent molecule 4 by a hybridization reaction, and the quenching probe 7 separates from the donor fluorescent probe 6, and thereby fluorescence is emitted by exciting the fluorescent molecule 4 modifying the donor fluorescent probe 6 modified with the fluorescent molecule 4 with excitation light.

In the present invention, "complementary" means that one nucleic acid sequence has a nucleic acid sequence capable of forming a double-stranded state with the other nucleic acid sequence, and does not necessarily have to be completely complementary, and several mismatched base pairs may be included in the case.

The fluorescent molecules used in the present invention are not particularly limited as long as they are molecules that generate fluorescence when being excited by a specific excitation light, but examples thereof include Alexa Fluor (registered trademark) series, ATTO series, Brilliant series, Chromeo (registered trademark) series, Bacteriochlorin series, FAM, TAMRA, Cy pigment series, FITC, HiLyte Fluor (registered trademark) series, Rhodamine series, Tide Fluor (registered trademark) series, iFluor (registered trademark) series, DY pigment series, and the like.

As the substrate used in the present invention, plate-shaped quartz, glass, silicon, single crystals such as calcium fluoride and sapphire, ceramics, resin materials, and the like, formed in a rectangular shape when viewed from above, can be used. Examples of resin materials include cycloolefin polymer (COP), cyclic olefin copolymer (COC), polycarbonate, acrylic resin, polyethylene resin, and the like, which are excellent in optical properties and chemical and thermal stability. The shape of the substrate when viewed from above may be any shape. In the present invention, since fluorescence obtained by irradiation of excitation light that excites fluorescent molecules from an opposite side to the solid-phase surface of the substrate is measured from the opposite side to the solid-phase surface of the substrate opposite, the substrate used in the present invention preferably uses a material that transmits excitation light that excites the fluorescent molecules and fluorescence obtained by irradiation of the excitation light.

Next, the solid-phase surface of the substrate to which the target and the capture molecule are binding is held so that the solid-phase surface of the substrate is in contact with a solution containing the light-absorbing substance.

For example, when a target in a sample solution is modified with a fluorescent molecule, and a capture molecule that specifically binds to the target is immobilized on a solid-phase surface of a substrate, the target modified with a fluorescent molecule in the sample solution binds to the capture molecule immobilized on the solid-phase surface of the substrate. The solid-phase surface of the substrate to which the target modified with this fluorescent molecule and the capture molecule have bound is held so that it is in contact with a solution containing the light-absorbing substance.

For example, when a capture molecule that specifically binds to a target in a sample solution is modified with a fluorescent molecule, and the target is immobilized on a solid-phase surface of a substrate, the capture molecule modified with a fluorescent molecule in the sample solution binds to the target immobilized on the solid-phase surface of the substrate. A solid-phase surface of a substrate to which the capture molecule modified with this fluorescent molecule and the target have bound is held so that the solid-phase surface of the substrate is in contact with a solution containing the light-absorbing substance.

Examples of the method of holding a substrate so that a solid-phase surface of the substrate is in contact with a solution containing the light-absorbing substance include a method of disposing a substrate in a container holding the solution so that the solid-phase surface of the substrate is in contact with the solution, a method of injecting the solution into a container, integrally produced with a substrate to have a solid-phase surface of the substrate therein, so that the solid-phase surface of the substrate is in contact with the solution, and the like. When the solution is injected into a container, integrally manufactured to have the solid-phase surface of the substrate inside the container, so that the solid-phase surface of the substrate is in contact with the solution, it is preferable that the container into which the solution is injected have an injection port through which the solution is injected and a structure capable of sealing the injection port after injection. In addition, the light-absorbing substance is added to the container in advance, and when a sample solution containing a target or a capture molecule is injected into a container, the light-absorbing substance may be added to the sample solution, and the light-absorbing substance may be added to the sample solution containing the target or the capture molecule and then injected into the container.

A timing of adding a light-absorbing substance to a sample solution may be any stage before irradiating a fluorescent molecule with excitation light, may be a stage of sample solution preparation or a stage before binding of a target and a capture molecule on a solid-phase surface of a substrate, and may be a stage after the binding of a target and a capture molecule on a solid-phase surface of a substrate.

By modifying a target or a capture molecule with a fluorescent molecule, and holding a solid-phase surface of a substrate to which the target and the capture molecule are binding so that the solid-phase surface of the substrate is in contact with a solution containing a light-absorbing substance that absorbs excitation light that excites the fluorescent molecule, when the solution is irradiated with excitation light that excites the fluorescent molecule in the solution, the light-absorbing substance contained in the solution can absorb excitation light trying to pass through the solution and suppress transmission of the excitation light. By suppressing the transmission of excitation light through a solution, it is possible to suppress excitation of a fluorescent molecule free in the solution, to suppress generation of fluorescence from the solution, and to reduce background light. In addition, when a substance that absorbs fluorescence emitted from a fluorescent molecule is used as a light-absorbing substance, even when the fluorescence substance in the solution is excited by excitation light and emits fluorescence, the light-absorbing substance can absorb the fluorescence emitted from the solution, and thereby it is possible to suppress the generation of fluorescence from the solution and to reduce the background light.

In addition, during a preparation of a solution containing a target, residual molecules such as proteins and sugars derived from specimens contained in a sample solution extracted from specimens containing living organisms and microorganisms may be included in the solution containing the target. Even in that case, since a light-absorbing substance can suppress generation of excitation or fluorescence of the residual molecules, even when it is applied to a device for target detection which does not originally require washing, background light due to fluorescence generated from the residual substance can be reduced, and detection sensitivity can be further improved.

The light-absorbing substance is not particularly limited as long as it has optical properties of absorbing excitation light that excites fluorescent molecules or light of a wavelength of fluorescence emitted from fluorescent molecules. Although it is appropriately selected according to the wavelength of fluorescence generated by fluorescent molecules used in the present invention, pigments used for coloring paints, inks, cosmetics, foods, and the like are used. Pigments has various types of particles, including metals such as gold and silver, oxides such as iron oxide, nitrides, and organic polymers, and can be used as a light-absorbing substance by being selected based on light absorption properties of a material itself, colored particles, and optical properties of light absorption by surface coloring. In addition, metal nanoparticles can be used as a light-absorbing substances because it has surface plasmon resonance occurring at a specific wavelength due to interactions between electrons on a particle surface and light, resulting in strong attenuation of light.

Specific examples of the light-absorbing substance used in the present invention include, for example, iron oxides (Fe₂O₃, Fe₂O₄), gold nanoparticles, silver nanoparticles, black-colored silica particles, and black-colored polymer particles such as copolymers of styrene and acrylic acid, and the like, when Cy3 (registered trademark) is used as a fluorescent molecule. The light-absorbing substance may be in a dry state or in a solution state.

When the light-absorbing substance is a substance that absorbs excitation light, a light-absorbing substance that passes through a solid phase and reduces scattering of excitation light that enters a solution containing the light-absorbing substance is preferred. This is because the light-absorbing substance that reduces scattering of excitation light that enters a solution containing the light-absorbing substance can further suppress a phenomenon in which light passing through the solution is scattered by the light-absorbing substance to extend an optical path length, which causes extra excitation of fluorescent molecules in the solution and increases fluorescence.

This phenomenon is a phenomenon which expresses a logarithm of a ratio of an incident light to a transmitted light that has passed through a substance as an absorbance, is similar to a phenomenon in which Modified Lambert-Beer law that has considered an effect of scattering formulates that there is a proportional relationship with the concentration and the optical path length with respect to Lambert-Beer law, which is a law that formulates it, and when a scattering substance is present, an increase in absorbance proportional to the concentration of the substance and the linear optical path length in a direction of an optical axis cannot be obtained. The scattering phenomenon of light by substances differs depending on a particle size, and is represented by geometrical optics approximation in the case of substances having a particle size much larger than the wavelength, Mie scattering in the case of substances having a particle size of about the wavelength, and Rayleigh scattering in the case of substances having a particle size sufficiently smaller than the wavelength. In the present invention, since a light-absorbing substance is added and dispersed in a solution, it is considered that Mie scattering or Rayleigh scattering occurs. It is known that a total scattering intensity of Mie scattering varies depending on a particle size and increases in proportion to the square to the sixth power of a particle size. In addition, it is known that the total scattering intensity of Rayleigh scattering increases in proportion to the sixth power of a particle size. For the reasons described above, in the present invention, it is preferable that the particle size of a light-absorbing substance is as small as possible.

When a light-absorbing substance is a substance that absorbs fluorescence, fluorescence from a fluorescent molecule near a contact surface between a solution and a solid-phase surface is faster than fluorescence from a fluorescent molecule inside the solution, and it reaches a detector installed on an opposite side to the solid-phase surface of a substrate. Since it is preferable that the fluorescence generated near the contact surface between the solution and the solid phase be absorbed by the light-absorbing substance without being scattered on the solution side and reaching the detector side, a light-absorbing substance that reduces the scattering of fluorescence is preferred.

In addition, since a light-absorbing substance is added into the solution, it is desirable that it be stably dispersed during fluorescence measurement and it do not cause unevenness in the solution. Dispersion stability of particles is measured by various measuring methods such as visual observation, measurement of transmitted light intensity change, and measurement of scattered light intensity change. For example, a difficulty of precipitation can be estimated based on parameters of centrifugation conditions for precipitation of a substance by a centrifuge, and the dispersion stability can be estimated. Polymer particles preferably have a particle size of less than 800 nm and parameters required for centrifugal separation are 10,000×g for 20 minutes, which makes them difficult to precipitate and excellent in dispersibility. It is preferable that particles have a particle size of 200 nm or less when the light-absorbing substance is a silica particle, a particle size of 100 nm or less in the case of an iron oxide particle, and a particle size of 15 nm or less in the case of gold nanoparticles because they are excellent in dispersibility.

Moreover, in the present invention, a light-absorbing substance is preferably hydrophilic in order to be added and dispersed in a solution. When a light-absorbing substance is hydrophobic, it is preferable to modify the surface to be hydrophilic, or to hydrophilize it by introducing surface functional groups such as carboxyl groups and sulfone groups, coating it with oxides, and chemically modifying it with hydrophilic polymers such as PEG, PEO, and dextran, and the like.

When a light-absorbing substance is added to a solution before binding a target and a capture molecule, it is preferable that the light-absorbing substance to be added do not adsorb the target or the capture molecule. For example, by modifying surfaces of particles of the light-absorbing substance with a hydrophilic polymer such as PEG, it is possible to suppress non-specific adsorption of the light-absorbing substance to the target or the capture molecule. In addition, by adding a blocking agent such as BSA that suppresses non-specific adsorption of biomolecules to a solution containing the light-absorbing substance, it is possible to suppress non-specific adsorption of the light-absorbing substance to the target or the capture molecule.

Next, fluorescence obtained by irradiation of excitation light that excites a fluorescent molecule from an opposite side to a solid-phase surface of the substrate is measured from the opposite side to the solid-phase surface of the substrate.

A method of measuring fluorescence obtained by irradiation of excitation light that excites a fluorescent molecule from an opposite side to a solid-phase surface of the substrate is not particularly limited as long as fluorescence emitted from a fluorescent molecule can be measured. However, examples thereof is, for example, a method of performing measurement by using a camera and binding a fluorescence image generated from a fluorescent molecule onto a detection element of the camera.

As an excitation light source, for example, a laser light source that emits laser light with a single wavelength or its expanded light, a light source consisting of a light emitting diode (LED), a lamp that emits white light, a combination of an LED and a wavelength filter, or the like can be used.

As a camera used for measurement, EM-CCD, digital CMOS, and the like which are characterized by high sensitivity as well as color and monochrome CCD and CMOS cameras can be used. In addition, a combination of a photodiode or the like, which is a single detector disposed one-to-one with a spot, may also be used.

The fluorescence image obtained by the target measurement method of the present invention can acquire images before and after binding of a target and a capture molecule at the same spot. For this reason, it is not affected by variations in light intensity between solid phases and between spots. Moreover, the amount of change in fluorescence can be calculated based on the fluorescence images before and after binding, and the number of bound molecules can be calculated. The amount of change in fluorescence may be calculated by using an average light intensity of an entire spot or the amount of change in fluorescence of each pixel of a spot image.

Next, a target measurement device of the present invention will be described. The target measurement device of the present invention can be used for the target measurement method of the present invention.

The target measurement device of the present invention is a target measurement device used when a target contained in a sample is measured, and is a target measurement device that includes a substrate in which the target or the capture molecule is immobilized on a solid-phase surface, and a container to which a light-absorbing substance that absorbs excitation light that excites a fluorescent molecule modifying a bound product of the target and the capture molecule, of fluorescence emitted from the fluorescent molecule is added, and which is capable of holding a solution containing the other of the target and the capture molecules and the light-absorbing substance while in contact with the solid-phase surface of the substrate.

Examples of the target measurement device of the present invention include a target measurement device in which a capture molecule is immobilized on a solid-phase surface of a substrate, and a solution containing a target modified by a fluorescent molecule is supplied to a container, a target measurement device in which a target is immobilized on a solid-phase surface of a substrate and a solution containing a capture molecule modified with a fluorescent molecule is supplied to a container, a target measurement device in which a capture molecule modified with a fluorescent molecule is immobilized on a solid-phase surface of a substrate, and a solution containing the target is supplied to a container, a target measurement device in which either one of a target and a capture molecule is immobilized on a solid-phase surface of a substrate and a solution containing the other of the target and the capture molecule and a fluorescent molecule binding to a bound body of the target and the capture molecule is supplied to a container, and the like. Examples of a target and a capture molecule include those described above.

FIG. 4 is a diagram which shows an example of a configuration of the target measurement device of the present invention. FIG. 4 shows an example of a DNA microarray on which a DNA probe is immobilized as a solid phase.

In the target measurement device of the present invention, a target or a capture molecule is immobilized on a solid-phase surface of a substrate. In FIG. 4, the DNA probe 1 having a sequence complementary to a specific nucleic acid sequence serving as a target is immobilized on the DNA microarray 5 serving as a substrate. The target measurement device of the present embodiment modifies the target 3 with the fluorescent molecule 4, and holds the container 32 capable of holding a target solution with light-absorbing substance added 35 containing the target 3 modified with the fluorescent molecule 4 and a light-absorbing substance that absorbs excitation light 33 that excites the fluorescent molecule 4 or fluorescence 34 emitted from the fluorescent molecule 4 while the target solution with light-absorbing substance added 35 is brought into contact with a solid-phase surface of the DNA microarray 5 on which the DNA probe 1 is immobilized.

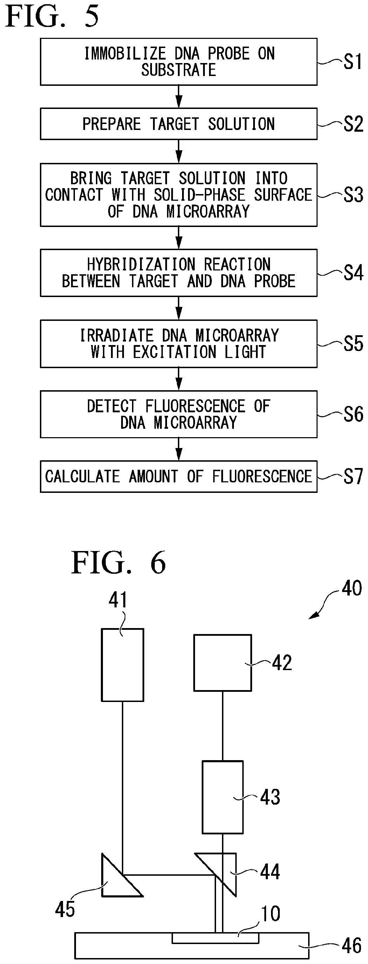

Next, a principle and an operation procedure for detecting the target 3 using a target measurement device will be described based on the target measurement device shown in FIG. 4. FIG. 5 is a flowchart which shows the operation procedure for detecting the target 3 using the target measurement device shown in FIG. 4.

First, the DNA probe 1 modified with the fluorescent molecule 4 is immobilized on a DNA spot 30 in a substrate (step S1). Next, the target 3 in a sample is modified with the fluorescent molecule 4, and a target solution is prepared (step S2). The target 3 having a specific nucleic acid sequence may be amplified during the preparation of the target solution. A timing for confirming whether the target 3 is present in the sample is not limited to after completion of the amplification, and may be during the amplification. When the target 3 is amplified, modification of the target 3 with the fluorescent molecule 4 may be performed after the amplification is confirmed, and the processing may proceed to step S3 described below only when the amplification is confirmed. As a method of confirming whether the target 3 is present, electrophoresis, antigen-antibody reaction, mass spectrometry, real-time PCR, and the like can be appropriately used.

Next, the prepared target solution is supplied to the container 32 which is a container to which a light-absorbing substance is added and in which a DNA microarray on which the DNA probe 1 is immobilized is disposed, and the target solution is brought into contact with the solid-phase surface of the DNA microarray 5 (step S3). The light-absorbing substance added to the container 32 is added to the target solution when the target solution is supplied to the container 32, and becomes the target solution with light-absorbing substance added 35.

After the target solution with light-absorbing substance added 35 is brought into contact with the solid-phase surface of the DNA microarray 5 on which the DNA probe 1 is immobilized, the target 3 modified with the fluorescent molecule 4 and the DNA probe 1 immobilized on the DNA microarray 5 are subjected to a hybridization reaction (step S4). According to this hybridization reaction, the target 3 binds to the DNA probe 1, and the fluorescent molecule 4 modifying the target 3 is captured in the DNA spot 30 on which the DNA probe 1 is immobilized.

After the hybridization reaction, irradiation of the excitation light 33 that excites the fluorescent molecule 4 is performed from an opposite side to the solid-phase surface of the DNA microarray 5 (step S5).

Next, the fluorescence 34 generated from the fluorescent molecule 4 that modifies the target 3 binding to the DNA probe 1 immobilized on the DNA microarray 5 is detected from the opposite side to the solid-phase surface of the DNA microarray 5 (step S6). For example, a fluorescence image generated from the fluorescent molecule 4 is acquired using a fluorescence reading device 40. Next, an amount of fluorescence is calculated based on the acquired fluorescence image (step S7).

When the irradiation of the excitation light 33 that excites the fluorescent molecule 4 is performed from the opposite side to the solid-phase surface of the DNA microarray 5, which is a substrate, fluorescence is emitted from the fluorescent molecule 4 that modifies the target 3.

When the light-absorbing substance is a substance that absorbs the excitation light 33 that excites the fluorescent molecule 4, the target solution with light-absorbing substance added 35 is irradiated with the excitation light 33 for exciting the fluorescent molecule 4. However, since the target solution with light-absorbing substance added 35 contains a light-absorbing substance that absorbs the excitation light 33 that excites the fluorescent molecule 4, excitation of the fluorescent molecule 4 of the target 3 modified with an unreacted fluorescent molecule 4 that does not hybridize with DNA probe 1, which is free in the solution 35, can be suppressed. In addition, when the light-absorbing substance is a substance that absorbs the fluorescence 34 emitted from the fluorescent molecule 4, the target solution with light-absorbing substance added 35 is irradiated with the excitation light 33 for exciting the fluorescent molecule 4. However, since the target solution with light-absorbing substance added 35 contains a light-absorbing substance that absorbs the fluorescence 34 emitted from the fluorescent molecule 4, generation of fluorescence from the fluorescent molecule 4 of the target 3 modified with an unreacted fluorescent molecule 4 that does not hybridize with the DNA probe 1, which is free in the solution 35, can be suppressed. As a result, since the solution 35 can suppress emission of fluorescence, fluorescence generated from the target 3 binding to a solid phase can be measured in the presence of a solution containing the target 3 without washing the solid phase. In addition, real-time measurement during the hybridization reaction is also possible.

Moreover, according to the target measurement method of the present invention, it is possible to calculate the number of molecules of the target 3 subjected to the hybridization reaction based on the amount change in fluorescence of the fluorescent molecule 4 before and after the hybridization reaction. For example, a hybridization reaction is performed using a standard solution of the target 3 having a known number of molecules, the amount of change in fluorescence of the fluorescent molecule 4 before and after the reaction is measured, and a calibration curve showing a relationship between the number of molecules and the amount of change in fluorescence is created in advance. Based on this calibration curve and the amount of change in fluorescence of the fluorescent molecule 4 before and after the hybridization reaction using a sample, it is possible to calculate the number of molecules of the target 3 subjected to the hybridization reaction. A light-absorbing substance is added to the solution in step S2 or step S3 to measure the amount of change in fluorescence of the fluorescent molecule 4 before and after the hybridization reaction.

Next, a method for manufacturing a target measurement device according to the target measurement method of the present invention will be described.

### (1) Solution preparation

First, a solution containing a target or a capture molecule that specifically binds to the target is prepared, and a concentration of the target or the capture molecule is adjusted. For example, when the target is DNA with a specific nucleic acid sequence and the capture molecule is a DNA probe with a sequence complementary to the specific nucleic acid sequence of the target, a DNA probe solution is prepared and a concentration of the DNA probe solution is adjusted.

### (2) Immobilization on solid-phase surface

The target or the capture molecule is immobilized on a solid-phase surface of a substrate. For example, when a DNA probe is immobilized on the solid-phase surface, the DNA probe 1 is spotted on the solid-phase surface using a spotter or the like, and the DNA probe 1 is immobilized on the solid-phase surface. The solid-phase surface is immersed in a blocking solution, and unreacted active functional groups are inactivated.

An area on the solid-phase surface on which the target or the capture molecule is spotted may be divided into blocks in units of a predefined number. A target solution is added to a target measurement device for each block. In addition, an image of the target measurement device is often acquired for each block. In other words, a block can be referred to as an image acquisition area.

### (3) Washing

Next, the solid-phase surface is washed, surplus targets or capture molecules that have not been immobilized are removed, and a washing liquid is also removed. A substrate produced by the procedure as above is appropriately stored until use under an environment suitable for properties of the substrate and the target or the capture molecule immobilized on the substrate, such as light shielding, temperature, and humidity conditions.

### (4) Disposition of substrate in container that holds solution containing light-absorbing substance

The substrate on which a target of a capture molecule is immobilized, obtained in (3), is disposed in the container so that the solid-phase surface on which the target or the capture molecule is immobilized is on a side of the container to hold a solution containing a light-absorbing substance. The substrate may be integrated with the container, or the substrate and the container may be separated so that the substrate can be disposed in the container when the target is measured. When the substrate is integrated with the container, it is preferable that the container has an injection port through which a solution can be injected and a structure capable of sealing the injection port after injection.

### (5) Addition of light-absorbing substance to container

A light-absorbing substance is added to the container prepared in (4). The light-absorbing substance can be in a dry state or a liquid state. By adding a light-absorbing substance to the container in advance, when a solution containing a target or a capture molecule modified with a fluorescent molecule is supplied to the container, the light-absorbing substance can be added to the solution. The light-absorbing substance may be added to the container before the substrate is disposed in the container or after the substrate is disposed in the container. When the light-absorbing substance is added after the substrate is disposed in the container, the light-absorbing substance can be added from the injection port provided in the container.

Next, a target measurement apparatus of the present invention will be described. The target measurement apparatus of the present invention includes the target measurement device of the present invention and a fluorescence reading device for measuring the fluorescence amount of fluorescent molecule from the target measurement device.
FIG. 6 is an example of a configuration diagram which shows the target measurement apparatus of the present invention. Since the target measurement apparatus of the present embodiment acquires images before and after binding of a target of the target measurement device 10 and a capture molecule, after acquiring an image before the binding, the temperature control stage increases the temperature of the solid-phase surface of the target measurement device 10 to allow a binding reaction to proceed, and an image after the binding is obtained after the temperature is again lowered to a room temperature. For example, when the DNA probe 1 is immobilized on the solid-phase surface of the substrate, the target 3 modified with the fluorescent molecule 4 is subjected to a hybridization reaction with the DNA probe 1, and images before and after the hybridization reaction are acquired.

The temperature control stage preferably has shaking, rotation of the target measurement device, or a stirring function of vortex mixer, and the like during the reaction between the target and the capture molecule to promote binding between the target and the capture molecule.

In an optical system of the fluorescence reading device 40, laser light emitted from the laser light source 41 is reflected by a dichroic mirror 44 via the mirror 45 and irradiates the solid-phase surface of the target measurement device. The irradiated light becomes the excitation light 33 for the fluorescent molecule 4 on the solid-phase surface of the target measurement device 10, the fluorescent molecule 4 is excited, and the fluorescent molecule 4 emits the fluorescence 34.

The fluorescence emitted from the solid-phase surface of the target measurement device 10 passes through the dichroic mirror 44, and a fluorescence image is formed and detected on a detection element of a CCD camera 42 via an image forming optical system 43. Here, to prevent the excitation light 33 from leaking into the fluorescence 34, a bandpass filter matching an excitation light wavelength may be installed on a side of the excitation light 33, and a bandpass filter matching a fluorescence wavelength to be detected may be installed on a side of the fluorescence 34.

A fluorescence image obtained by the target measurement apparatus of the present invention can acquire images before and after binding of a target and a capture molecule at the same spot. For this reason, it is not affected by variations in light intensity between solid phases and between spots. In addition, an amount of change in fluorescence can be calculated based on fluorescence images before and after a binding reaction, and the number of molecules subjected to the binding reaction can be calculated. The amount of change in fluorescence may be calculated by using an average light intensity of an entire spot or the amount of change in fluorescence of each pixel of a spot image.

The target measurement apparatus of the present invention may also include a computer that controls the CCD camera 42, an arithmetic device that calculates a light intensity of an image, and a recording device that stores the image and the light intensity.

The target measurement apparatus of the present invention is not limited to the embodiments described above. Since the target measurement apparatus of the present invention detects fluorescence from a surface opposite to an immobilized surface of a detection spot on the solid-phase surface, it can be used with fluorescence microscopes, confocal microscopes, evanescent fluorescence detectors, thin-film oblique illumination microscopes, sheet illumination microscopes, structured illumination microscopes, multiphoton excitation microscopes, and the like.

Next, a target measurement kit of the present invention will be described. The target measurement kit of the present invention can be used for the target measurement method of the present invention.

The target measurement kit of the present invention is a target measurement kit used when a target contained in a sample is measured, and includes a substrate on which either one of the target and the capture molecule is immobilized on a solid-phase surface, a container capable of holding a solution containing the other of the target and the capture molecule while in contact with the solid-phase surface of the substrate, and a light-absorbing substance which absorbs excitation light that excites fluorescent molecules modifying a bound product of the target and the capture molecule or fluorescence emitted from the fluorescent molecules.

A light-absorbing substance may be added to a sample solution when the sample solution containing a target or a capture molecule is prepared. As a timing for adding the light-absorbing substance to the sample solution may be at any stage before irradiating a fluorescent molecule with the excitation light, and it may also be, for example, a stage of preparing a sample solution, a stage before binding of a target and a capture molecule on a solid phase, or a stage after binding of a target and a capture molecule on a solid phase.

A light-absorbing substance may be added in advance to the container. The light-absorbing substance may be in a dry or liquid state. By adding the light-absorbing substance to the container in advance, when a solution containing a target or a capture molecule modified with a fluorescent molecule is supplied to the container, the light-absorbing substance can be added to the solution.

For example, a capture molecule may be immobilized on a solid-phase surface of the substrate, the container may be a container capable of holding a solution containing a target modified with a fluorescent molecule and the light-absorbing substance while in contact with a surface of the substrate, a target may be immobilized on a solid-phase surface of the substrate, and the container may also be a container capable of holding a solution containing a capture molecule modified with a fluorescent molecule and the light-absorbing substance while in contact with the solid-phase surface.

In addition, the capture molecule modified with the fluorescent molecule is immobilized on the solid-phase surface of the substrate, and the container may be a container capable of holding a solution containing the target and the light-absorbing substance while in contact with the solid-phase surface.

In addition, either one of the target and the capture molecule is immobilized on the solid-phase surface of the substrate, and the container may also be a container capable of holding a solution containing the other of the target and the capture molecule, a fluorescent molecule binding to the bound body of the target and the capture molecule, and the light-absorbing substance while in contact with the solid-phase surface of the substrate.

Examples of a target and a capture molecule include the target and the capture molecule described above. Examples of the target measurement kit of the present invention include, for example, a kit in which a target having a specific nucleic acid sequence serves as a target and a DNA probe having a sequence complementary to the specific nucleic acid sequence serves as a capture molecule, and the like.

The target measurement kit of the present invention may include the target measurement device of the present invention.

In the target measurement kit of the present invention, examples of a substrate, a light-absorbing substance, and a container include those mentioned above. The target measurement kit of the present invention may further include a standard solution, a necessary buffer solution, a product instruction, and the like necessary for quantifying a target.

Next, a method of using the target measurement kit of the present invention will be described using a kit containing the target measurement device shown in FIG. 4 as an example. In this target measurement kit, the target is DNA having a specific nucleic acid sequence, and the capture molecule is a DNA probe having a sequence complementary to the specific nucleic acid sequence. The DNA probe is immobilized on the solid-phase surface of the DNA microarray, and the target is modified by fluorescent molecule.

The target measurement kit of the present embodiment is a container capable of holding the DNA microarray 5 on which the DNA probe 1 is immobilized and a target solution with light-absorbing substance added 35, and may include the container 32 which holds the solution 35 in contact with the solid-phase surface of the DNA microarray 5 on which the DNA probe 1 is immobilized, and a light-absorbing substance is added to the container 32.

First, the target 3 in a sample is modified with the fluorescent molecule 4 and a target solution is prepared. The target 3 having a specific nucleic acid sequence may be amplified when the target solution is prepared. Next, the target solution is supplied to the container 32 and is brought into contact with a solid phase surface of the DNA microarray 5. The light-absorbing substance added to the container 32 is added to the target solution when the target solution is supplied to the container 32, and becomes the target solution with light-absorbing substance added 35. After the target solution with light-absorbing substance added 35 is brought into contact with the solid-phase surface of the DNA microarray 5 on which the DNA probe 1 is immobilized, the target 3 modified with the fluorescent molecule 4 and the DNA probe 1 immobilized on the DNA microarray 5 are subjected to a hybridization reaction. According to this hybridization reaction, the target 3 binds to the DNA probe 1, and the fluorescent molecule 4 modifying the target 3 is captured in the DNA spot 30 on which the DNA probe 1 is immobilized.

After the hybridization reaction, the excitation light 33 that excites the fluorescent molecule 4 is emitted from the opposite side to the solid-phase surface of the DNA microarray 5.

Next, the fluorescence 34 generated from the fluorescent molecule 4 that modifies the target 3 binding to the DNA probe 1 immobilized on the DNA microarray 5 is detected from the opposite side to the solid-phase surface of the DNA microarray 5. For example, a fluorescence image generated from fluorescent molecule 4 is acquired by the fluorescence reading device 40. Then, the amount of fluorescence is calculated based on the acquired fluorescence image.

An application scope of the present invention is not limited to the embodiments described above. The present invention can be widely applied to a target measurement method, a target measurement device, a target measurement apparatus, and a target measurement kit that measure a target included in a sample.

The target measurement method, the target measurement device, the target measurement apparatus, and the target measurement kit of the present invention may be used for dry image measurement in a fluorescent molecule light intensity measurement, an in-liquid observation of fluorescent molecule light intensity of a biochip, a real-time observation in continuous reaction, and the like. Specifically, for example, it can be used for discrimination of bacterial species according to a gene or polymer analysis, discrimination of oncogenes, discrimination of animals and plants, inspection of intestinal bacteria, and the like.

In addition, the target measurement method, the target measurement device, the target measurement apparatus, and the target measurement kit of the present invention are also applicable to solid phase methods such as labeled antibody methods used in clinical examinations and the like. For example, a fluorescence in situ hybridization (FISH) method of measuring fluorescence, which detects expression of specific chromosomes or genes in tissues or cells using a fluorescence substance, can be exhibited as an example. In addition, they are also applicable to a fluorescence immunoassay (FIA) method, which measures an antigen-antibody reaction using a fluorescence luminescent substance such as europium as a label, and an indirect fluorescence antibody (IFA) method, which measures a serum (antibody) reaction in which pathogens that serve as antigens are labeled with a fluorescence substance.

### [Example]

Hereinafter, the present invention will be described in more detail based on examples, but the present invention is not limited by these.

### (Example 1)

A reduction effect of background light by adding a light-absorbing substance was confirmed. A Cy3 (registered trademark) molecule was adjusted to a concentration of 0.3 to 3,000 nM as the fluorescent molecule 4 in a container, and a transparent glass substrate was disposed in the container. A fluorescence image was obtained by irradiation of the excitation light 33 of 532 nm through the transparent glass substrate.

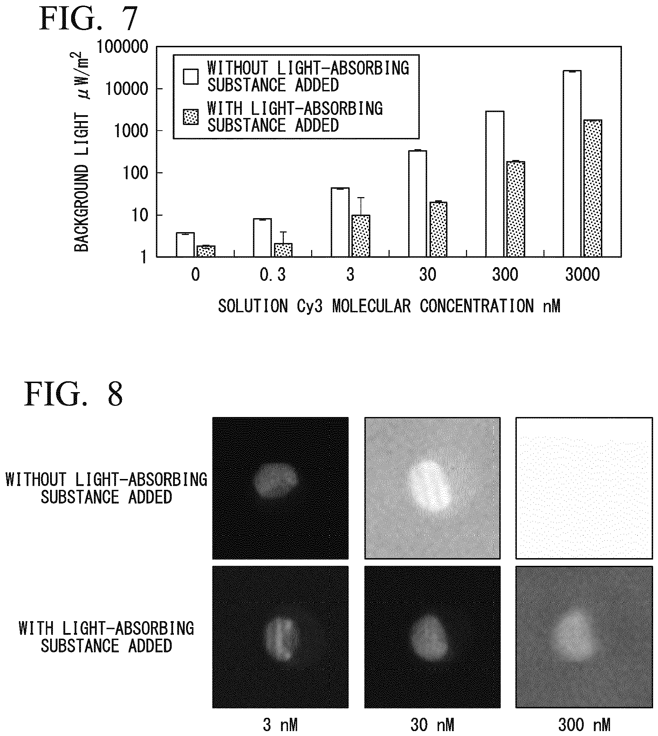

FIG. 7 shows results of calculating the light intensity of background light based on the acquired fluorescence images when a light-absorbing substance is added to a solution in a container and when it is not added. In addition, Table 1 also shows a background light ratio (the light intensity of background light when a light-absorbing substance is not added/the light intensity of background light when a light-absorbing substance is added) when the concentration of a fluorescent molecule is changed. The light-absorbing substance used was dextran-coated iron oxide (Fe2O3) with a particle size of 50 nm, and was added to the solution to have a concentration of 50 mg/ml.

**[Table 1]**

| | | | | | | |
|---|---|---|---|---|---|---|
| Solution Cy3 molecule concentration | 0 | 0.3 | 3 | 30 | 300 | 3000 |
| Background light ratio (with/without light-absorbing substance added) | 2.0 | 4.0 | 4.3 | 17 | 15 | 15 |

As shown in FIG. 7 and Table 1, background light can be reduced from 1/4 to 1/17 according to a Cy3 molecule concentration, and the reduction effect increases when the background light is high. However, when genomic DNA is extracted from microorganisms, it is known that the fluorescence of a solution is about 10 µW/m², and since the effect is obtained even in a solution with a background light of about 10 µW/m², it is known that there is the effect even in fluorescence generated by exciting the solution caused by molecules other than the target 3 brought from the sample. In addition, it is known that background light can be reduced to 1/2 even in a state of water that does not contain Cy3 molecules. However, it is considered that this is because reflection and self-fluorescence at the bottom of the container is decreased due to a fact that the excitation light 33 does pass through the solution, and a secondary effect was confirmed.

In addition, a difference in a way to view a spot by reducing the background light was examined. FIG. 8 is a spot image of a spot on which Cy3 molecule-modified synthetic DNA is immobilized on a substrate, obtained by a fluorescence reading device in a solution with the Cy3 molecule concentration of 3 to 300 nM with an exposure time of 1 second. When the Cy3 molecule concentration is 30 nM or more, the background light can be reduced to 1/10 or more, so it was known that spot observation is possible even when the fluorescence emitted by the solution is high.

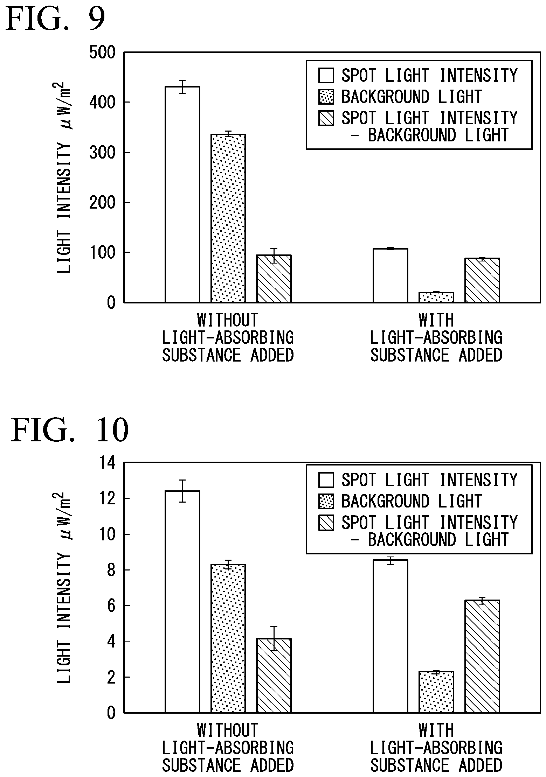

FIG. 9 shows a relationship between spot light intensity and background light when the Cy3 molecule concentration is 30 nM. An amount of fluorescence emitted by the fluorescent molecule 4, captured by the DNA probe 1 of a spot, is calculated based on a difference between the light intensity of the spot and the background light, but as shown in FIG. 9, the light intensity did not change depending on the presence or absence of a light-absorbing substance add, and the detection signal did not decrease by adding a light-absorbing substance. In addition, when a ratio of the spot light intensity to the background light is set to S/N, the S/N was 5.3 when a light-absorbing substance was added whereas the S/N was 1.3 when a light-absorbing substance was not added, which means that the S/N was improved by 4.2 times.

### (Example 2)

The DNA microarray 5 was prepared by disposing a plurality of DNA probes 1 non-modified with the fluorescent molecule 4 on a substrate, and applicability of the addition of a light-absorbing substance was confirmed as follows in spot observation by hybridization of target DNA modified with a Cy3 molecule.

The target DNA modified with a Cy3 molecule was prepared to have a concentration of 0.25 nM in a container, a light-absorbing substance was added under the same conditions as in Example 1, and the DNA microarray 5 was disposed. After incubating for 30 minutes at 60°C and 5 rpm, the DNA probe 1 and the target DNA were subjected to a hybridization reaction. After returning to room temperature, the fluorescence image of a spot was acquired by the fluorescence reading device, and the light intensity was calculated. The results are shown in FIG. 10.

As shown in FIG. 10, when a light-absorbing substance was added, the fluorescence of the target modified by an unreacted Cy3 molecule, which is free in a solution, was reduced, and the background light was reduced to 1/3.7. At this time, if the ratio of the spot light intensity to the background light is set to S/N, the S/N was 3.7 when a light-absorbing substance was added, whereas the S/N was 1.5 when a light-absorbing substance was not added, which means that the S/N was improved by 2.5 times. Compared to when a light-absorbing substance was not added, the difference between the spot light intensity and the background light when a light-absorbing substance was added did not cause a decrease in light intensity, and thus it can be known that a light-absorbing substance does not interfere with the hybridization reaction between the DNA probe 1 and the target 3.

### [Reference Signs List]

1 DNA probe
2 Detection sequence
3 Target
4 Fluorescent molecule
5 DNA microarray
6 Donor fluorescent probe
7 Quenching probe
8 Quenching substance
10 Target measurement device
30 DNA spot
32 Container
33 Excitation light
34 Fluorescence
35 Target solution with light-absorbing substance added
40 Fluorescence reading device
41 Laser light source
42 CCD camera
43 Image forming optical system
44 Dichroic mirror
45 Mirror
46 Stage

## Claims

1. A target measurement method for measuring a target contained in a sample, comprising:
when a solution containing a light-absorbing substance that absorbs excitation light that excites a fluorescent molecule or fluorescence emitted from the fluorescent molecule is in contact with a solid-phase surface of a substrate that is provided with a bound product of the target and a capture molecule specifically binding to the target which is modified with a fluorescent molecule, measuring a fluorescence obtained by irradiation of the excitation light from an opposite side to the solid-phase surface of the substrate from the opposite side to the solid-phase surface of the substrate.

2. The target measurement method according to claim 1, further comprising:
modifying the target with the fluorescent molecule; and
obtaining the bound product by binding the target to the capture molecule immobilized on the solid-phase surface.

3. The target measurement method according to claim 1, further comprising:
modifying the capture molecule with the fluorescent molecule; and
obtaining the bound product by binding the capture molecule to the target immobilized on the solid-phase surface.

4. The target measurement method according to claim 1, further comprising:
modifying the capture molecule with the fluorescent molecule; and
obtaining the bound product by binding the target to the capture molecule immobilized on the solid-phase surface.

5. The target measurement method according to claim 1, further comprising:
obtaining the bound product by supplying either one of the target and the capture molecule immobilized on the solid-phase surface and the fluorescent molecule to the other of the target and the capture molecule.

6. The target measurement method according to any one of claims 1 to 5, wherein
the target is a nucleic acid with a specific nucleic acid sequence,
the capture molecule is a detection probe having a sequence complementary to the specific nucleic acid sequence, and
the target is subjected to a hybridization reaction with the detection probe to obtain the bound product.

7. A target measurement device used when a target contained in a sample is measured, comprising:
a substrate on which either one of the target and a capture molecule specifically binding to the target is immobilized on a solid-phase surface; and
a container to which a light-absorbing substance that absorbs excitation light that excites a fluorescent molecule that modify a bound product of the target and the capture molecule or fluorescence emitted from the fluorescent molecule is added, and which is capable of holding a solution containing the other of the target and the capture molecule and the light-absorbing substance while in contact with the solid-phase surface of the substrate.

8. The target measurement device according to claim 7, wherein
the capture molecule is immobilized on the solid-phase surface, and
a solution containing the target modified with the fluorescent molecule is supplied to the container.

9. The target measurement device according to claim 7, wherein
the target is immobilized on the solid-phase surface, and
a solution containing the target modified with the fluorescent molecule is supplied to the container.

10. The target measurement device according to claim 7, wherein
the capture molecule, which is modified with the fluorescent molecule, is immobilized on the solid-phase surface, and
a solution containing the target is supplied to the container.

11. The target measurement device according to claim 7, wherein
either one of the target and the capture molecule is immobilized on the solid-phase surface, and
a solution containing the other of the target and the capture molecule and the fluorescent molecule binding to a bound body of the target and the capture molecule is supplied to the container.

12. The target measurement device according to any one of claims 7 to 11, wherein
the target is a target having a specific nucleic acid sequence, and
the capture molecule is a detection probe having a sequence complementary to the specific nucleic acid sequence.

13. A target measurement apparatus comprising:
the target measurement device according to any one of claims 7 to 12; and
a fluorescence reading device configured to measure an amount of fluorescence from the target measurement device.

14. A target measurement kit used when a target contained in the sample is measured, comprising:
a substrate on which either one of the target and a capture molecule specifically binding to the target is immobilized on a solid-phase surface,
a container capable of holding a solution containing the other of the target and the capture molecule while in contact with the solid-phase surface of the substrate, and
a light-absorbing substance configured to absorb excitation light that excites a fluorescent molecule modifying a bound product of the target and the capture molecule or fluorescence emitted from the fluorescent molecule.

15. The target measurement kit according to claim 14, wherein the light-absorbing substance is added to the container in advance.

16. The target measurement kit according to claim 14 or 15, wherein
the capture molecule is immobilized on the solid-phase surface, and
a solution containing the target modified with the fluorescent molecule and the light-absorbing substance is held in the container.

17. The target measurement kit according to claim 14 or 15, wherein
the target is immobilized on the solid-phase surface, and
a solution containing the capture molecule modified with the fluorescent molecule and the light-absorbing substance is held in the container.

18. The target measurement kit according to claim 14 or 15, wherein
the capture molecule modified with the fluorescent molecule is immobilized on the solid-phase surface, and
a solution containing the target and the light-absorbing substance is held in the container.

19. The target measurement kit according to claim 14 or 15, wherein
either one of the target and the capture molecule is immobilized on the solid-phase surface, and
a solution containing the other of the target and the capture molecule, the fluorescent molecule binding to a bound body of the target and the capture molecule, and the light-absorbing substance is held in the container.

20. The target measurement kit according to any one of claims 14 to 19, wherein
the target is a target having a specific nucleic acid sequence, and
the capture molecule is a detection probe having a sequence complementary to the specific nucleic acid sequence.
